# EUROPEAN PATENT SPECIFICATION

(11) **EP 0 547 068 B1**
(45) Date of publication and mention of the grant of the patent: **25.09.1996**
(21) Application number: 91914242.2
(22) Date of filing: 24.07.1991
(51) Int. Cl.: A61M 5/158, A61M 5/32

(54) **A CANNULA**
KANULE
CANULE

(30) Priority: 07.09.1990 NO 903910
(43) Date of publication of application: 23.06.1993
(73) Proprietor: PREISS, Otto T., N-0393 Oslo 3 (NO); Heimreid, Bent, N-3942 Skjelsvik (NO)
(72) Inventor: HEIMREID, Bent, N-3942 Skjelsvik (NO)
(74) Representative: Reinhard - Skuhra - Weise & Partner
(86) International application number: NO9100104
(87) International publication number: WO9204062

(56) References cited:
- DE-A- 2 005 519
- DE-C- 346 472
- GB-A- 1 298 707
- SE-C- 135 689
- US-A- 2 187 259
- US-A- 2 524 713
- US-A- 2 601 580
- US-A- 4 368 730
- US-A- 4 642 101
- US-A- 4 808 170

## Description

The present invention relates to a cannula, preferably an intravenous cannula of the kind that has a bevelled point forming a cutting edge.

At present, there is a variety of cannulae on the market, however, they only differ in details as regards connection with tubing, medicament wells, and the like.

We shall refer to US-PS 4.231.367 in the name of VIGGO AB as the most relevant art in the field.

This cannula is designed for the intravenous administration of medicaments and comprises, in general terms, a housing with flaps provided on the under side for contact with the skin, a medicament well with a lid, optional gripping means and means for connection to conventional intravenous equipment, and further comprises an insertion needle unit having a needle which extends through a soft catheter tube. The needle is of a conventional type, ground in the same way as a regular cannula needle where the cutting edge which is to penetrate the skin first and thereafter the vein wall is located on the portion of the needle circumference, which upon insertion, because of the shape of the cannula unit, must lie closest to the skin.

As examples of known technology, we refer to EP 0 238 419 A3 and EP 0 271 775 A3, DE-OS 24 34 618, GP-PS 1 298 707, and US-PS 2 748 769.

All cited documents disclose straight cannulae or cannulae which are slightly arched at the point, with the cutting edge of the cannula extending along the periphery of the cannulae tube, and with the cannula, additionally, being equipped with auxiliary openings or with a two-piece cannula opening, this to provide a certain turbulence when injecting.

There are also cannulae on the market the cutting edge of which is displaced by grinding to a point between the periphery and the light opening.

Experiments proved that the cannula according to the invention results in a smaller penetration or wound area than known cannulae.

This was confirmed in tests on patients, up to 90 % of whom said on inquiry that piercing with the cannula according to the invention caused less discomfort than piercing with cannulae of known technology. The tests were made with masked cannulae, one of each kind and one on each arm of the same patient.

Another advantage which is achieved with the cannula according to the invention is that the cannula opening will face the stream when inserted into the vein, which will provide a more rapid "backflow" and, thus, confirmation that the cannula is in place.

It is an object of the present invention to further improve the prior art as it has been described herein above.

The invention provides an intravenous cannula unit comprising a catheter unit and an insertion needle with a bevelled point which forms a cutting edge and a bevelled surface, the catheter unit, in addition to a medicament well, attachment flaps and connecting means to intravenous equipment, comprising a flexible catheter tube, where the insertion needle extends through the catheter tube and projects from the free and thereof, characterized in that the insertion needle is fixed in a position on the intravenous cannula unit so that the light opening and the bevelled surface of the insertion needle faces the skin surface prior to penetration of the skin and the vein wall.

Other advantages of the invention will appear from the following disclosure with reference to the accompanying drawings, in which:
- Figure 1: shows a known cannula according to US-PS 4 231 367 with a cannula tube 1 and a point 2;
- Figure 2: shows an enlarged portion of the point area of Figure 1:
- Figure 3: shows a convertional cannula in a position for penetration;
- Figure 4: shows the situation in case of unintentional penetration of the opposite vene wall;
- Figure 5: shows the unintended situation occuring when the cannula tube is wrongly placed;
- Figure 6: shows an unintentional perforation of the cannula tube by the pont of the cannula;
- Figure 7: shows the general position of the point of the cannula according to the invention (compare with Figure 2);
- Figure 8: corresponds to Figure 3, but shows the invention;
- Figure 9: basically corresponds to Figure 4, but shows the advantage achieved by the invention;
- Figure 10 and Figure 11: show a preferred embodiment of the invention (compare with Figure 7);
- Figure 12: shows the preferred embodiment and corresponds to Figure 9;
- Figure 13: shows the preferred embodiment and corresponds to Figure 6;

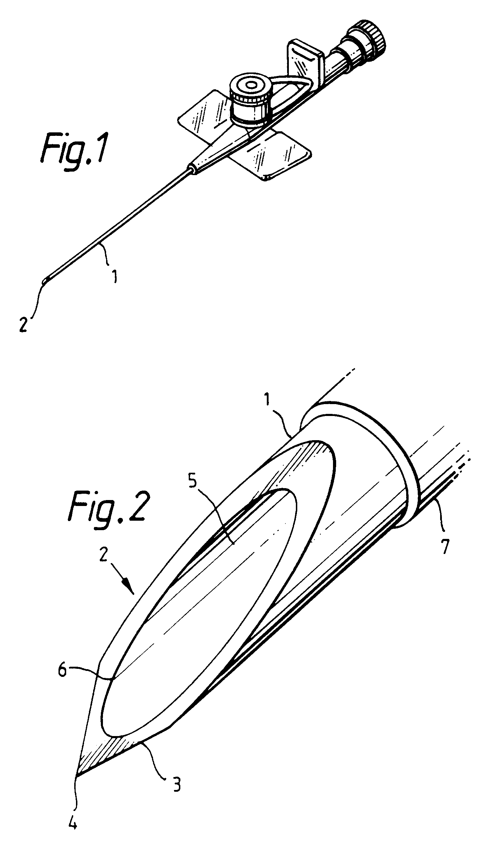

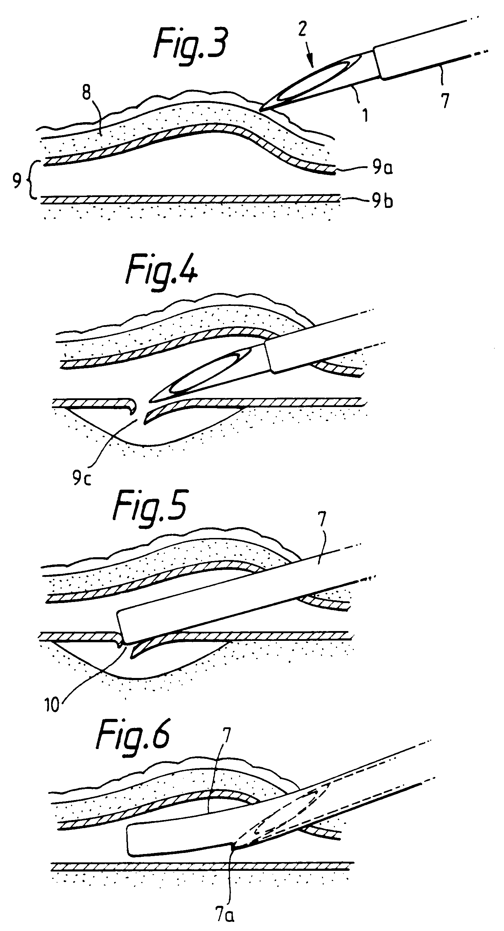

As mentioned above all cannulae on the market at present are of the kind as shown in Figure 1, designed as shown in Figure 2 with the edge of the point being provided in the part of the cannula wall that is intended to lie in contact with the skin when used.

This is shown diagrammatically in Figure 2, and the accidents that may occur when the cannula is used are illustrated in Figures 3 - 6.

In Figure 3 cannula 1 with point area 2 and the associated plastic tube 7 is positioned to penetrate the skin 8 and vein 9 by also piercing one vein wall 9b.

Figure 4 shows what may happen if the cannula is inserted too steeply or forcefully. The opposite vein wall 9c is punctured, which will cause a hazard of blood oozing out of the vein with a consequent haematoma. The patient, thus, must be pierced once more.

In use, blood and medicament may then ooze out of opening 9c, so that there is also an accumulation of liquid outside the vein with further hazard of injuries to tissue and necrosis, see Figure 5.

Figure 6 shows a situation which may occur due to the fact that the point of the cannula is moved inside the cannula tube, the latter may be punctured at 7a.

As mentioned above, one of the objects of the invention is to remedy the shortcomings of known technology, and according to the general design of this invention, this is achieved in that the cutting edge 3 of the cannula point 2 along the portion of the cannula wall, when in use, is intended to face the skin and is fixed to the intravenous cannula in the fixed position prior to penetrating the vein.

Generally, the cutting edge is achieved by simple angular cutting of the cannula; however it is possible both on conventional cannulae and on cannulae according to the invention to sharpen the cutting cannula edge, as indicated in Figure 2, so as to provide a regular point 4 instead of the present oval arch.

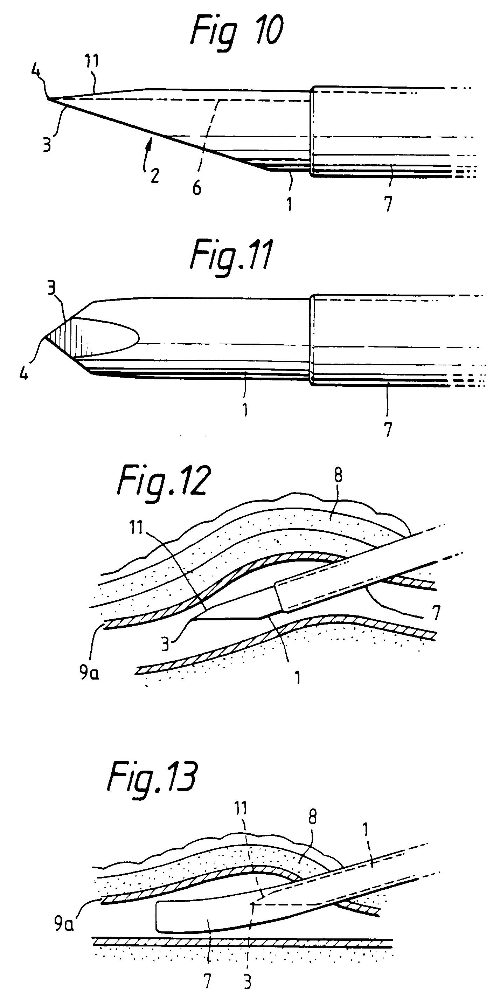

The cannula according to the invention is illustrated in Figures 8 and 9 with Figure 8 corresponding to Figure 3 immediately before the skin is punctured.

In Figure 9 the situation shown is immediately after cannula 1 with plastic tube 7 has penetrated the skin 8 and one vein wall 9a. It will appear that by placing the cutting edge according to the invention a more flat "underside" of the point area 2 is achieved, which will substantially reduce the hazard of puncturing the other vein wall 9b, as shown in Figure 4.

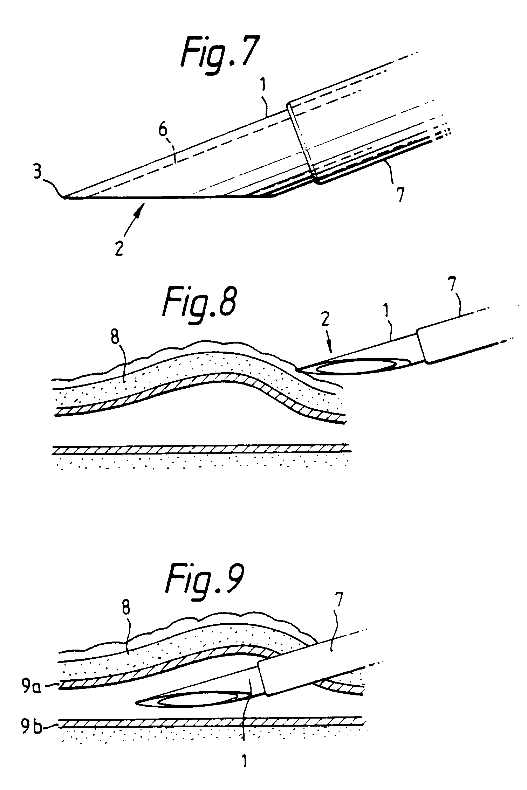

The embodiment shown in Figures 7 - 9 represents an essential improvement as compared to known technology, as illustrated in Figures 3 - 6.

There is, however, a certain though strongly reduced hazard of unintentional penetration of a vein wall upon insertion of the cannula, also in connection with the object of the present invention. This is shown directly in Figure 4, but even after successful insertion a puncture may occur on the side where the point of the cannula is placed.

The preferred embodiment of the invention, as mentioned above, should solve this problem too, and this is the case with the embodiment generally shown in Figure 10.

It is achieved by reshaping the point area 2 to move the cutting edge 3 of the cannula from the outer diameter to the inner diameter or light opening, indicated by numeral 6 in Figures 7 and 10.

By moving the cutting edge 3 the hazard of unintentionally puncturing the vein wall is substantially reduced, as there is provided a further surface 11 as will appear, e.g. from Figure 12. Said surface may slide against the vein wall and preventing the point from getting caught in the vein wall and unintentionally puncturing the wall.

According to the invention as disclosed above a cannula is achieved in a simple and inexpensive manner, and safety in operation and use is enhanced without sacrificing the safety of known cannulae and, additionally, without causing essentially increased costs.

The invention, thus, represents an essential improvement as regards operation and use of cannulae in connection with puncturing and cannulation of veins.

## Claims

1. An intravenous cannula unit comprising a catheter unit and an insertion needle (1) with a bevelled point (2) which forms a cutting edge and a bevelled surface, the catheter unit, in addition to a medicament well, attachment flaps and connecting means to intravenous equipment, comprising a flexible catheter tube (7), where the insertion needle (1) extends through the catheter tube (7) and projects from the free and thereof,
**characterized in**
that the insertion needle (1) is fixed in a position on the intravenous cannula unit so that the light opening and the bevelled surface (6) of the insertion needle (1) faces the skin surface prior to penetration of the skin (8) and the vein wall (9a).

## Patentansprüche

1. Intravenös anwendbare Kanüleneinheit mit einer Kathetereinheit und einer Einführnadel (1) mit einer abgeschrägten Spitze (2), die eine Schnittkante und eine abgeschrägte Fläche bildet, wobei die Kathetereinheit, zusätzlich zu einer Öffnung für Medikamente, Ansatzklappen und einer Verbindungseinrichtung an eine Einrichtung zur intravenösen Versorgung, ein flexibles Katheterrohr (7) aufweist und die Einführnadel (1) durch das Katheterrohr (7) reicht und aus dessen freiem Ende hervorsteht,
dadurch **gekennzeichnet**,
daß die Einführnadel (1) auf der intravenösen Kanüleneinheit derart fixiert ist, daß die kleine Öffnung und die abgeschrägte Fläche (6) der Einführnadel (1) vor dem Durchstechen der Haut (8) und der Venenwand (9a) zur Hautoberfläche hin ausgerichtet sind.

## Revendications

1. Unité de canule intraveineuse, comprenant une unité de cathéter et une aiguille d'insertion (1) comportant une pointe biseautée (2) qui forme une arête coupante et une surface biseautée, l'unité de cathéter comprenant, en sus d'un réservoir de médicament, de languettes de fixation et de moyens de branchement à un équipement intraveineux, un tube cathéter flexible (7), l'aiguille d'insertion (1) s'étendant dans le tube cathéter (7) et faisant saillie à l'extrémité libre de ce tube cathéter, caractérisée en ce que l'aiguille d'insertion (1) est immobilisée dans une position sur l'unité de canule intraveineuse de façon que la lumière intérieure (6) et la surface biseautée de l'aiguille d'insertion (1) soient disposées face à la surface de la peau avant perforation de la peau (8) et de la paroi (9a) de la veine.
